# EUROPEAN PATENT SPECIFICATION

(11) **EP 0 731 708 B1**
(45) Date of publication and mention of the grant of the patent: **05.09.2001**
(21) Application number: 95902258.3
(22) Date of filing: 30.11.1994
(51) Int. Cl.: A61K 35/78, A61K 7/48

(54) **EXTRACT FROM THE LEAVES OF GINKGO BILOBA**
GINKGO BILOBA BLÄTTER EXTRAKT
EXTRAIT DE FEUILLES DE GINGKO BILOBA

(30) Priority: 03.12.1993 IE 930939
(43) Date of publication of application: 18.09.1996
(73) Proprietor: MONTANA LIMITED, County Cork (IE)
(72) Inventor: O'REILLY, Joseph, County Cork (IE)
(74) Representative: Schütte, Gearoid
(86) International application number: IE9400059
(87) International publication number: WO9515172

(56) References cited:
- DATABASE WPI Section Ch, Week 8810, Derwent Publications Ltd., London, GB; Class D22, AN 88-067422 & JP,A,63 021 061 (DAICEL CHEM IND KK) 28 January 1988
- PATENT ABSTRACTS OF JAPAN vol. 012, no. 139 (C-491) 27 April 1988 & JP,A,62 255 433 (NIPPON GREEN UEEBU KK) 7 November 1987

## Description

The invention relates to a new extract from the leaves of *Gingko biloba* and its method of preparation.

Certain extracts from the leaves of *Gingko biloba* are widely used for the therapy of peripheral and cerebral arterial circulatory disturbances. Various processes for preparing such extracts are described for example in DE-B-1767098, DE-B 2117429, EP-A 0 324 197, EP-A 330 567 and EP-A 0 436 129.

According to one aspect of the invention there is provided an extract from the leaves of *Gingko biloba* containing at least 25% glycolipids by weight and less than 50 ppm of ginkgolic acids. In a preferred embodiment of the invention the extract includes at least 1% by weight cerebrosides.

In one embodiment of the invention the extract contains at least 35% glycolipids, preferably approximately 40% glycolipids. In this case preferably the glycolipids comprise a mixture of monogalactodiglycerides, digalactodiglycerides and cerebrosides. Preferably the weight ratio of monogalactodiglycerides to digalactodiglycerides is from 1:1 to 3:1, most preferably approximately 5:3.

In another embodiment of the invention the extract contains less than 50% glycolipids and cerebrosides. In this case preferably the extract contains from 30% to 45% glycolipids and cerebrosides. Preferably the glycolipids are predominantly digalactodiglycerides. In this case preferably the weight ratio of glycolipids to cerebrosides is from 2 : 1 to 6 : 1, most preferably approximately 4 : 1. Typically the cerebrosides include galactocerebroside which is preferably present in an amount of from 3% to 12%, most preferably 3% to 10% by weight.

The invention also provides a method for preparing a glycolipid extract from *Gingko biloba* leaves comprising the steps of :
extracting the leaves with an organic solvent;
separating the extract from the leaves;
cooling the extract to precipitate a lipid fraction;
recovering the lipid fraction from the extract;
mixing the lipid fraction with an alcohol;
removing insoluble material;
extracting the alcoholic solution;
treating the alcoholic solution with a first extracting medium to remove neutral lipids and ginkgolic acids;
treating the alcoholic solution with a second extracting medium to extract glycolipids;
purifying the second medium containing the glycolipids; and
removing the second medium to provide a concentrated glycolipid extract.

Preferably the concentrated alcoholic solution is dissolved in an organic solvent and the solution is applied to a chromatographic column, the alcoholic solution being treated with the first extracting medium by passing the first medium through the column. Typically the first medium is an organic solvent, preferably a mixture of acetone and toluene.

Preferably the concentrated alcoholic solution is treated with the second extracting medium by passing the second medium through the column. Typically the second extracting medium is a mixture of an organic solvent and an alcohol, preferably a mixture of acetone and ethanol.

In one embodiment of the invention the organic solvent used to extract the leaves is acetone/water. Preferably after recovery from the extract the lipid fraction is washed and then further separated. Typically the alcohol with which the lipid fraction is mixed is ethanol.

In a preferred arrangement the medium containing the glycolipids is purified by treating with charcoal and filtering the treated extract to remove the charcoal.

Preferably the second medium is removed by evaporation.

In another aspect the constituents of the extract of the invention have a similar polarity to that of glycolipids.

The invention also provides a topical composition including an extract of the invention.

The invention will be more clearly understood from the following description thereof, given by way of example only.

In general terms in the process of the invention, the leaves of *Gingko biloba* are first extracted with an organic solvent such as acetone/water and the resulting solution is concentrated, for example, by evaporation and cooled. The solution is then filtered and a C₁-C₃ alcohol such as ethanol is added to the precipitate. The solution is agitated and again filtered. The filtrate is adjusted with water and may be extracted with a suitable solvent such as petroleum spirit and/or hexane/heptane. The aqueous alcoholic phase is then evaporated to dryness and an organic solvent, preferably acetone/toluene, is added.

In one embodiment of the invention the organic solvent is a 5% to 30%, most preferably 15% acetone/solvent mixture.

In another embodiment of the invention the organic solvent is a 40% to 60%, most preferably 50% acetone/solvent mixture.

The resulting solution is filtered, typically concentrated, and then purified by chromatographic separation using a first medium, typically acetone/toluene. The glycolipid fraction is removed using a second medium typically comprising acetone/ethanol. The glycolipid fraction is then purified, for example by treating with charcoal, filtered to remove the charcoal, and then evaporated for complete removal of the acetone/toluene solvents.

The invention also provides an extract in which the constituents have a similar polarity to that of the glycolipids.

The process will be described in further detail in the following Examples.

### EXAMPLE 1

### Step 1

100 kg of finely milled *Gingko biloba* leaves were extracted with 800 1 of a 60% acetone water mixture in a counter current extraction unit at a temperature of 55-60°C.

The extract was separated from the leaves and was then concentrated by evaporation.

### Step 2

The concentrate was cooled to 8-10°C and was retained at this temperature for 3 hours.

The lipids which precipitated were recovered by decanting.

The recovered lipids were then dissolved in an agitated solution of 2.9 kg of acetone and 0.7 kg of demineralised water. A further 30 kg of demineralised water was then added.

The solution was continuously agitated and cooled to 8-10°C and was retained for a minimum of 3 hours before being decanted.

### Step 3

The wet decanted lipids from Step 2 were added to 200 kg of ethanol and agitated for 60 minutes.

Insoluble material was removed by decanting.

### Step 4

The ethanolic solution from Step 3 was adjusted to 12-15% water.

The solution was extracted twice with 140 1 of petroleum distillate (boiling range 60-80°C).

The ethanol-water phase was evaporated to greater than 85% dry extract.

### Step 5

The extract from Step 4 was dissolved in 15% acetone/toluene.

This solution was filtered and was then applied to 30 kg of silica in a packed column. 800 1 of 15% acetone/toluene were pumped through the columns. The column was then flushed with 200 1 of 7.5% ethanol/acetone.

### Step 6

The ethanol/acetone fraction was purified by addition of 1.1 kg Charcoal to the ethanol/acetone fraction and the solution was agitated for 30 minutes.

The solution was filtered and the solvent removed by evaporation.

0.98 kg of a green brown oil having the following characteristics are obtained :-

| | |
|---|---|
| Glycolipids | 40% |
| consisting of Monogalactodiglycerides | 25% and |
| Digalactodiglycerides | 15% |
| Ginkgolic Acids | 39 ppm |
| Galactocerebroside | 6% |

### EXAMPLE 2

### Step 1

70 kg of finely milled *Gingko biloba* leaves were extracted with 560 1 of a 60% acetone water mixture in a counter current extraction unit at a temperature of 55-60°C.

The extract was separated from the leaves and was then evaporated to reduce the acetone content to less than 3%.

### Step 2

The concentrate was cooled to 8-10°C and was retained at this temperature for 3 hours .

The lipids which precipitated were recovered by decanting.

The recovered lipids were dissolved in an agitated solution of 2.0 kg of acetone. A further 20 kg of demineralised water was then added.

The solution was continuously agitated and cooled to 8-10°C and was retained for a minimum of 3 hours before being decanted.

### Step 3

The wet decanted lipids from Step 2 were added to 30 kg of methanol and agitated for 60 minutes.

Insoluble material was removed by decanting. The filtrate was evaporated to remove methanol.

### Step 4

10 kg of acetone was added to the concentrate from Step 3 and agitated for 1 hour. The resulting solution was filtered and the filtrate evaporated.

### Step 5

The extract from Step 4 was dissolved in 15% acetone/toluene.

This solution was filtered and was then applied to 8.5 kg of silica in a packed column. 60 1 of 15% acetone/toluene were pumped through the columns. The column was then flushed with 17 1 of 7.5% ethanol/toluene.

### Step 6

0.24 kg of Charcoal was added to the ethanol/acetone fraction and the solution was agitated for 30 minutes.

The solution was filtered and the solvent removed by evaporation.

0.19 kg of a green brown oil having the following characteristics are obtained :-

| | |
|---|---|
| Glycolipids | 27% |
| consisting of Monogalactodiglycerides | 20% and |
| Digalactodiglycerides | 7% |
| Ginkgolic Acids | 30 ppm |
| Galactocerebroside | 3.5% |

### EXAMPLE 3

### Step 1

70 kg of finely milled *Gingko biloba* leaves were extracted in with 600 1 of a 60% acetone water mixture in a counter current extraction unit at a temperature of 55-60°C.

The extract was separated from the leaves and was then concentrated by evaporation.

### Step 2

The concentrate was cooled to 8-10°C and was retained at this temperature for a minimum 3 hours.

The lipids which precipitated were recovered by decanting.

The recovered lipids were then dissolved in an agitated solution of 2.2 kg of acetone and 0.5 kg of demineralised water. A further 22 kg of demineralised water was then added.

The solution was continuously agitated and cooled to 8-10°C and was retained for a minimum of 3 hours before being decanted.

### Step 3

The wet decanted lipids from Step 2 were added to 55 kg of ethanol and agitated for 60 minutes.

Insoluble material was removed by decanting.

The ethanol-water phase was evaporated to greater than 85% dry extract.

### Step 4

The extract from Step 3 was dissolved in 50% acetone/toluene.

This solution was filtered and was then applied to 18.5 kg of silica in a packed column. 150 1 of 50% acetone/toluene were pumped through the columns. The column was then flushed with 100 1 of 7.5% ethanol/acetone.

### Step 5

The solution was concentrated and rechromotographed as above.

The ethanol/acetone fraction was purified by addition of 0.06 kg Charcoal to the ethanol/acetone fraction and the solution was agitated for 30 minutes.

The solution was filtered and the solvent removed by evaporation.

0.26 kg of a green brown paste having the following characteristics are obtained :-

| | |
|---|---|
| Glycolipids | 33% |
| consisting of Monogalactodiglycerides | 1% and |
| Digalactodiglycerides | 31% |
| Ginkgolic Acids | not detected |
| Galactocerebroside | 10% |

### EXAMPLE 4

### Step 1

5 kg of finely milled *Gingko biloba* leaves were extracted with 40 1 of a 60% acetone water mixture in a counter current extraction unit at a temperature of 50-60°C.

The extract was separated from the leaves and was then evaporated to reduce the acetone content to less than 3%.

### Step 2

The concentrate was cooled to 8-10°C and was retained at this temperature for 3 hours.

The lipids which precipitated were recovered by decanting.

The recovered lipids were dissolved in an agitated solution of 0.15 kg of acetone. A further 1.5 kg of demineralised water was then added.

The solution was continuously agitated and cooled to 8-10°C and was retained for a minimum of 3 hours before being decanted.

### Step 3

The wet decanted lipids from Step 2 were added to 2 kg of methanol and agitated for 60 minutes.

Insoluble material was removed by decanting. The filtrate was evaporated to remove methanol.

### Step 4

0.5 kg of acetone was added to the concentrate from Step 3 and agitated for 1 hour. The resulting solution was filtered and the filtrate evaporated.

### Step 5

The extract from Step 4 was dissolved in 20% acetone/toluene.

This solution was filtered and was then applied to 0.8 kg of silica in a packed column. 5.5 1 of 20% acetone/toluene were pumped through the columns. The column was then flushed with 1.6 1 of ethanol.

### Step 6

25 g of Charcoal was added to the ethanol fraction and the solution was agitated for 30 minutes.

The solution was filtered and the solvent removed by evaporation.

19 g of a green brown oil having the following characteristics are obtained :-

| | |
|---|---|
| Glycolipids | 42% |
| consisting of Monogalactodiglycerides | 29% |
| Digalactodiglycerides | 13% |
| Galactocerebroside | 3% |

It will be appreciated that while the extracts have been characterised using [technique] as containing glycolipids it may be possible that the major constituents have a polarity similar to that of glycolipids.

The extracts have shown activity in cosmetic and skin care applications.

The invention is not limited to the specific embodiments hereinbefore described, which may be varied in detail.

## Claims

1. Extract from the leaves of *Gingko biloba* containing at least 25% of glycolipids by weight and less than 50 ppm of ginkgolic acid.

2. An extract as claimed in claim 1 including at least 1% by weight cerebrosides.

3. An extract as claimed in claim 1 or 2 containing at least 35% glycolipids.

4. An extract as claimed in any of claims 1 to 3 containing approximately 40% glycolipids.

5. An extract: as claimed in any preceding claim wherein the glycolipids comprise a mixture of monogalactodiglycerides and digalactodiglycerides.

6. An extract as claimed in claim 5 wherein the weight ratio of monogalactodiglycerides to digalactodiglycerides is from 1:1 to 3:1, typically the ratio is approximately 5:3.

7. An extract as claimed in claim 1 or 2 wherein the extract contains less than 50% glycolipids and cerebrosides by weight, the extract may contain from 30% to 45% glycolipids and cerebrosides.

8. An extract as claimed in any of claims 3 to 7 wherein the glycolipids are predominantly digalactodi-glycerides.

9. An extract as claimed in claims 7 or 8 wherein the ratio of glycolipids to cerebrosides is from 2 : 1 to 6 : 1; typically approximately 4 : 1.

10. An extract as claimed in any of claims 2 to 9 wherein the cerebrosides include galactocerebroside, in this case the galactocerebroside may be present in an amount of from 3% to 12% by weight, typically in an amount of from 3% to 10% by weight.

11. An extract from the leaves of *Gingko biloba* selected from :-
an extract from the leaves of *Gingko biloba* containing 25% by weight of monogalactodiglycerides, 15% by weight digalactodiglycerides, 6% by weight cerebrosides and less than 50 ppm ginkolic acids;
an extract from the leaves of *Gingko biloba* containing 20% by weight of monogalactodiglycerides, 7% by weight digalactodiglycerides, 3.5% by weight cerebrosides and less than 50 ppm ginkolic acids;
an extract from the leaves of *Gingko biloba* containing 1% by weight monogalactodiglycerides, 31% by weight digalactodiglycerides, 10% by weight cerebrosides and less than 50 ppm ginkolic acid; or
an extract from the leaves of *Gingko biloba* containing 29% by weight monogalactodiglycerides, 13% by weight digalactodiglycerides, 3% by weight cerebrosides and less than 50ppm ginkolic acids.

12. A method for preparing a glycolipid extract from *Gingko biloba* leaves comprising the steps of:
extracting the leaves with an organic solvent;
separating the extract from the leaves;
cooling the extract to precipitate a lipid fraction;
recovering the lipid fraction from the extract;
mixing the lipid fraction with an alcohol;
removing insoluble material;
extracting the alcoholic solution;
treating the alcoholic solution with a first extracting medium to remove neutral lipids and ginkgolic acids;
treating the alcoholic solution with a second extracting medium to extract glycolipids;
purifying the second medium containing the glycolipids; and
removing the second medium to provide a concentrated glycolipid extract.

13. A method as claimed in claim 12 wherein the concentrated alcoholic solution is dissolved in an organic solvent and the solution is applied to a chromatographic column, the alcoholic solution being treated with the first extracting medium by passing the first medium through the column.

14. A method as claimed in claim 13 wherein the first medium is an organic solvent, typically the organic solvent is a mixture of acetone and toluene.

15. A method as claimed in claim 14 wherein the organic solvent is 5% to 30% acetone/toluene, typically the organic solvent is 15% acetone/toluene.

16. A method as claimed in claim 14 wherein the organic solvent is 40% to 60% acetone/toluene, typically the organic solvent is 50% acetone/toluene.

17. A method as claimed in any of claims 12 to 16 wherein the concentrated alcoholic solution is treated with the second extracting medium by passing the second medium through the column, typically the second extracting medium is a mixture of an organic solvent and an alcohol, preferably a mixture of acetone and ethanol.

18. A method as claimed in any of claims 12 to 17 wherein the organic solvent used to extract the leaves is acetone/water.

19. A method as claimed in any of claims 12 to 18 wherein after recovery from the extract the lipid fraction is washed and then further separated, preferably the alcohol with which the lipid fraction is mixed is ethanol.

20. A method as claimed in any of claims 12 to 19 wherein the medium containing the glycolipids is purified by treating with charcoal and filtering the treated extract to remove the charcoal.

21. A method as claimed in any of claims 12 to 20 wherein the second medium is removed by evaporation.

22. A glycolipid extract from *Gingko biloba* leaves whenever prepared by a method as claimed in any of claims 12 to 21.

23. An extract as claimed in any of claims 1 to 11 wherein the constituents have a similar polarity to that of glycolipids.

24. A topical composition including an extract as claimed in any of claims 1 to 11, claim 22 or claim 23.

## Patentansprüche

1. Extrakt aus den Blättern von *Ginkgo biloba*, der mindestens 25 Gew.-% Glycolipide und weniger als 50 ppm Ginkgolsäure enthält.

2. Extrakt nach Anspruch 1, der mindestens 1 Gew.-% Cerebroside einschließt.

3. Extrakt nach Anspruch 1 oder 2, der mindestens 35% Glycolipide enthält.

4. Extrakt nach einem der Ansprüche 1 bis 3, der ca. 40% Glycolipide enthält.

5. Extrakt nach einem der vorangehenden Ansprüche, worin die Glycolipide ein Gemisch aus Monogalactodiglyceriden und Digalactodiglyceriden umfassen.

6. Extrakt nach Anspruch 5, worin das Gewichtsverhältnis von Monogalactodiglyceriden zu Digalactodiglyceriden von 1:1 bis 3:1 beträgt, wobei das Verhältnis in der Regel ca. 5:3 beträgt.

7. Extrakt nach Anspruch 1 oder 2, worin der Extrakt weniger als 50 Gew.-% Glycolipide und Cerebroside enthält, wobei der Extrakt von 30% bis 45% Glycolipide und Cerebroside enthalten kann

8. Extrakt nach einem der Ansprüche 3 bis 7, worin die Glycolipide überwiegend Digalactodiglyceride sind.

9. Extrakt nach Ansprüchen 7 oder 8, worin das Verhältnis von Glycolipiden zu Cerebrosiden von 2:1 bis 6:1, in der Regel ca. 4:1 beträgt.

10. Extrakt nach einem der Ansprüche 2 bis 9, worin die Cerebroside Galactocerebrosid einschließen, wobei das Galactocerebrosid in diesem Fall in einer Menge von 3 Gew.-% bis 12 Gew.-%, in der Regel in einer Menge von 3 Gew.-% bis 10 Gew.-% vorliegen kann.

11. Extrakt aus den Blättern von *Ginkgo biloba*, ausgewählt aus: -
Einem Extrakt aus den Blättern von *Ginkgo biloba*, der 25 Gew.-% Monogalactodiglyceride, 15 Gew.-% Digalactodiglyceride, 6 Gew.-% Cerebroside und weniger als 50 ppm Ginkgolsäuren enthält;
einem Extrakt aus den Blättern von *Ginkgo biloba*, der 20 Gew.-% Monogalactodiglyceride, 7 Gew.-% Digalactodiglyceride, 3,5 Gew.-% Cerebroside und weniger als 50 ppm Ginkgolsäuren enthält;
einem Extrakt aus den Blättern von *Ginkgo biloba*, der 1 Gew.-% Monogalactodiglyceride, 31 Gew.-% Digalactodiglyceride, 10 Gew.-% Cerebroside und weniger als 50 ppm Ginkgolsäure enthält oder
einem Extrakt aus den Blättern von *Ginkgo biloba*, der 29 Gew.-% Monogalactodiglyceride, 13 Gew.-% Digalactodiglyceride, 3 Gew.-% Cerebroside und weniger als 50 ppm Ginkgolsäuren enthält.

12. Verfahren zur Herstellung eines Glycolipidextraktes aus *Ginkgo biloba*-Blättern, das die folgenden Schritte umfasst:
Extrahieren der Blätter mit einem organischen Lösungsmittel;
Trennen des Extraktes aus den Blättern;
Abkühlen des Extraktes zum Präzipitieren einer Lipidfraktion;
Rückgewinnen der Lipidfraktion aus dem Extrakt;
Mischen der Lipidfraktion mit einem Alkohol;
Entfernen von unlöslichem Material;
Extrahieren der alkoholischen Lösung;
Behandeln der alkoholischen Lösung mit einem ersten Extraktionsmedium zur Entfernung neutraler Lipide und Ginkgolsäuren;
Behandeln der alkoholischen Lösung mit einem zweiten Extraktionsmedium zum Extrahieren von Glycolipiden;
Reinigen des zweiten Mediums, das die Glycolipide enthält und
Entfernen des zweiten Mediums zum Vorsehen eines konzentrierten Glycolipidextraktes.

13. Verfahren nach Anspruch 12, worin die konzentrierte alkoholische Lösung in einem organischen Lösungsmittel aufgelöst wird und das Lösungsmittel auf eine chromatographische Säule aufgebracht wird, wobei die alkoholische Lösung mit dem ersten Extraktionsmedium behandelt wird, wobei das erste Medium durch die Säule geleitet wird.

14. Verfahren nach Anspruch 13, worin das erste Medium ein organisches Lösungsmittel ist, wobei das organische Lösungsmittel in der Regel ein Gemisch aus Aceton und Toluen ist.

15. Verfahren nach Anspruch 14, worin das organische Lösungsmittel 5% bis 30% Aceton/Toluen ist, wobei das organische Lösungsmittel in der Regel 15% Aceton/Toluen ist.

16. Verfahren nach Anspruch 14, worin das organische Lösungsmittel 40% bis 60% Aceton/Toluen ist, wobei das organische Lösungsmittel in der Regel 50% Aceton/Toluen ist.

17. Verfahren nach einem der Ansprüche 12 bis 16, worin die konzentrierte alkoholische Lösung mit dem zweiten Extraktionsmedium durch Durchleiten des zweiten Mediums durch die Säule behandelt wird, wobei das zweite Extraktionsmedium in der Regel ein Gemisch aus einem organischen Lösungsmittel und einem Alkohol, bevorzugt ein Gemisch aus Aceton und Ethanol ist.

18. Verfahren nach einem der Ansprüche 12 bis 17, worin das zum Extrahieren der Blätter verwendete organische Lösungsmittel Aceton/Wasser ist.

19. Verfahren nach einem der Ansprüche 12 bis 18, worin die Lipidfraktion nach der Rückgewinnung aus dem Extrakt gewaschen und dann weiter getrennt wird, wobei der Alkohol, mit dem die Lipidfraktion gemischt wird, bevorzugt Ethanol ist.

20. Verfahren nach einem der Ansprüche 12 bis 19, worin das Medium, das die Glycolipide enthält, durch Behandlung mit Aktivkohle und Filtrieren des behandelten Extraktes zur Entfernung der Aktivkohle gereinigt wird.

21. Verfahren, nach einem der Ansprüche 12 bis 20, worin das zweite Medium durch Eindampfung entfernt wird.

22. Glycolipidextrakt aus *Ginkgo biloba*-Blättern, wann immer er durch ein Verfahren nach einem der Ansprüche 12 bis 21 hergestellt wird.

23. Extrakt nach einem der Ansprüche 1 bis 11, worin die Bestandteile eine ähnliche Polarität wie die von Glycolipiden aufweisen.

24. Topische Zusammensetzung, einschließlich eines Extraktes nach einem der Ansprüche 1 bis 11, Anspruch 22 oder 23.

## Revendications

1. Extrait des feuilles de *Ginkgo biloba* contenant au moins 25% de glycolipides en poids et moins de 50 ppm d'acide ginkgolique.

2. Extrait selon la revendication 1, comportant au moins 1% en poids de cérébrosides.

3. Extrait selon la revendication 1 ou 2, contenant au moins 35% de glycolipides.

4. Extrait selon l'une quelconque des revendications 1 à 3, contenant approximativement 40% de glycolipides.

5. Extrait selon l'une quelconque des revendications précédentes, dans lequel les glycolipides comprennent un mélange de monogalactodiglycérides et de digalactodiglycérides.

6. Extrait selon la revendication 5, dans lequel le rapport pondéral des monogalactodiglycérides aux digalactodiglycérides est de 1:1 à 3:1, typiquement le rapport est d'environ 5:3.

7. Extrait selon la revendication 1 ou 2, dans lequel l'extrait contient moins de 50% de glycolipides et de cérébrosides en poids, l'extrait peut contenir de 30% à 45% de glycolipides et de cérébrosides.

8. Extrait selon l'une quelconque des revendications 3 à 7, dans lequel les glycolipides sont principalement des digalactodiglycérides.

9. Extrait selon la revendication 7 ou 8, dans lequel le rapport des glycolipides aux cérébrosides est de 2:1 à 6:1, typiquement environ de 4:1.

10. Extrait selon l'une quelconque des revendications 2 à 9, dans lequel les cérébrosides comportent du galactocérébroside, dans ce cas le galactocérébroside peut être présent en une quantité de 3% à 12% en poids, typiquement en une quantité de 3% à 10% en poids.

11. Extrait des feuilles de *Ginkgo biloba* choisi parmi :
un extrait des feuilles de *Ginkgo biloba* contenant 25% en poids de monogalactodiglycérides, 15% en poids de digalactodiglycérides, 6% en poids de cérébrosides et moins de 50 ppm d'acides ginkgoliques ;
un extrait des feuilles de *Ginkgo biloba* contenant 20% en poids de monogalactodiglycérides, 7% en poids de digalactodiglycérides, 3,5% en poids de cérébrosides et moins de 50 ppm d'acides ginkgoliques ;
un extrait des feuilles de *Ginkgo biloba* contenant 1% en poids de monogalactodiglycérides, 31% en poids de digalactodiglycérides, 10% en poids de cérébrosides et moins de 50 ppm d'acide ginkgolique; ou
un extrait des feuilles de *Ginkgo biloba* contenant 29% en poids de monogalactodiglycérides, 13% en poids de digalactodiglycérides, 3% en poids de cérébrosides et moins de 50 ppm d'acides ginkgoliques.

12. Méthode de préparation d'un extrait de glycolipides des feuilles de *Ginkgo biloba* comprenant les étapes :
d'extraction des feuilles par un solvant organique ;
de séparation de l'extrait depuis les feuilles ;
de refroidissement de l'extrait pour précipiter une fraction lipidique ;
de récupération de la fraction lipidique depuis l'extrait ;
de mélange de la fraction lipidique avec un alcool ;
d'élimination du matériau insoluble ;
d'extraction de la solution alcoolique ;
de traitement de la solution alcoolique par un premier milieu d'extraction pour éliminer les lipides neutres et les acides ginkgoliques ;
de traitement de la solution alcoolique par un deuxième milieu d'extraction pour extraire les glycolipides ;
de purification du deuxième milieu contenant les glycolipides ; et
d'élimination du deuxième milieu pour fournir un extrait de glycolipides concentré.

13. Méthode selon la revendication 12, dans laquelle la solution alcoolique concentrée est dissoute dans un solvant organique et la solution est déposée sur une colonne de chromatographie, la solution alcoolique étant traitée par le premier milieu d'extraction par passage du premier milieu à travers la colonne.

14. Méthode selon la revendication 13, dans laquelle le premier milieu est un solvant organique, typiquement le solvant organique est un mélange d'acétone et de toluène.

15. Méthode selon la revendication 14, dans laquelle le solvant organique est de l'acétone/toluène de 5% à 30%, typiquement le solvant organique est de l'acétone/toluène à 15%.

16. Méthode selon la revendication 14, dans laquelle le solvant organique est de l'acétone/toluène de 40% à 60%, typiquement le solvant organique est de l'acétone/toluène à 50%.

17. Méthode selon l'une quelconque des revendications 12 à 16, dans laquelle la solution alcoolique concentrée est traitée par le deuxième milieu d'extraction par passage du deuxième milieu à travers la colonne, typiquement le deuxième milieu d'extraction est un mélange d'un solvant organique et d'un alcool, préférablement un mélange d'acétone et d'éthanol.

18. Méthode selon l'une quelconque des revendications 12 à 17, dans laquelle le solvant organique utilisé pour extraire les feuilles est de l'acétone/eau.

19. Méthode selon l'une quelconque des revendications 12 à 18, dans laquelle, après récupération depuis l'extrait, la fraction lipidique est lavée et puis séparée davantage, préférablement l'alcool avec lequel la fraction lipidique est mélangée est l'éthanol.

20. Méthode selon l'une quelconque des revendications 12 à 19, dans laquelle le milieu contenant les glycolipides est purifié par traitement avec du charbon de bois et filtration de l'extrait traité pour éliminer le charbon de bois.

21. Méthode selon l'une quelconque des revendications 12 à 20, dans laquelle le deuxième milieu est éliminé par évaporation.

22. Extrait de glycolipides des feuilles de *Ginkgo biloba* tel que préparé par une méthode selon l'une quelconque des revendications 12 à 21.

23. Extrait selon l'une quelconque des revendications 1 à 11, dans lequel les constituants ont une polarité semblable à celle des glycolipides.

24. Composition topique comportant un extrait selon l'une quelconque des revendications 1 à 11, la revendication 22 ou la revendication 23.
